# EUROPEAN PATENT SPECIFICATION

(11) **EP 2 124 930 B1**
(45) Date of publication and mention of the grant of the patent: **13.10.2010**
(21) Application number: 07858187.3
(22) Date of filing: 27.12.2007
(51) Int. Cl.: A61K 31/34, A61P 1/00, A61P 29/00, A61P 43/00

(54) **ISOSORBIDE MONONITRATE DERIVATIVES FOR THE TREATMENT OF INTESTINAL DISORDERS**
ISOSORBID-MONONITRAT-DERIVATE ZUR BEHANDLUNG VON DARMERKRANKUNGEN
DÉRIVÉS DE MONONITRATE D'ISOSORBIDE POUR LE TRAITEMENT DE TROUBLES INTESTINAUX

(30) Priority: 28.12.2006 EP 06380338
(43) Date of publication of application: 02.12.2009
(73) Proprietor: LACER, S.A., 08025 Barcelona (ES)
(72) Inventor: REPOLLÉS MOLINER, José, E-08025 Barcelona (ES); PUBILL COY, Francisco, E-08025 Barcelona (ES); MOURELLE MANCINI, Marisabel, E-08025 Barcelona (ES)
(74) Representative: ABG Patentes, S.L.
(86) International application number: PCT/EP2007/064591
(87) International publication number: WO 2008/080955

(56) References cited:
- EP-A1- 1 120 419
- WO-A-2005/037842
- NALLET J P ET AL: "SYNTHESIS OF A SERIES OF HEXITOL AND AMINODEOXY.HEXITOL MONO-NITRATE DERIVATIVES CONTAINING A SULFUR GROUP AND PHARMACOLOGICAL EVALUATION OF ISOLATED RAT AORTAS" EUROPEAN JOURNAL OF ORGANIC CHEMISTRY, WILEY-VCH VERLAG, WEINHEIM, DE, no. 5, 1998, pages 933-943, XP001204497 ISSN: 1434-193X

## Description

The present invention relates to disulfide, sulfide, sulfoxide and sulfone derivatives of dianhydrohexite mononitrate of formula (I), tautomers, pharmaceutically acceptable salts, and solvates thereof for the prevention and/or treatment of intestinal disorders.

### BACKGROUND

Inflammatory bowel disease (IBD) is the generic term for a disease of an unknown cause that produces chronic inflammation or ulceration of the mucosa of the large and small intestine. This inflammatory bowel disease includes such diseases as ulcerative colitis and Crohn's disease.

The presently available medical treatments for IBD generally involve drug therapy directed towards the suppression of gastrointestinal inflammation. The most commonly used medicaments to treat IBD are anti-inflammatory drugs such as salicylates. The salycilate preparations may be effective in treating mild to moderate disease. Examples of salicylates include sulfasalazine, olsalazine and mesalamine. All of these medicaments are given orally in high doses for maximal therapeutic benefit. These medicaments are not without side effects including heartburn, nausea, vomiting, diarrhea and headache. People with more severe IBD can be treated with corticosteroids, such as prednisone and hydrocortisone, which are more potent and faster-acting than salicylates in the treatment of IBD, but are endowed with potential side effects. In IBD patients that do not response to salicylates or corticosteroids, medicaments that suppress the immune system are used. However, immunosuppressants cause increase risk of infection, renal failure, and may increase the need for hospitalization. Drugs like antidiarrheals, laxatives and pain relievers can be also given to help relieve symptoms. Since all the available medical treatments for IBD are rather unsatisfactory and often ineffective, there is currently a great need for novel drugs capable of treating IBD and preventing relapse.

Disulfide, sulfide, sulfoxide and sulfone derivatives of dianhydrohexite mononitrate have been used or evaluated in various studies related to different pathological conditions mediated by defects in the NO pathway, such as cardiovascular disorders [WO00/20420 and W02005/037842]. However, with all of these studies, there has been no recognition that these compounds are capable of being effective in the treatment of intestinal disorders, such as intestinal inflammatory.

### BRIEF DESCRIPTION OF THE INVENTION

The inventors have found surprisingly that compounds of formula (I), and specially 2-acetylthioisosorbide-5-mononitrate, have potential therapeutic effect against intestinal disorders, more specifically intestinal inflammation.

The new application of these compounds is based on the results obtained by *in vivo* experiments in animals subjected to different stimulus such as intestinal inflammation, wherein it has been observed that administration of compounds of formula (I) reduces significantly the adverse effect. Therefore, these compounds have a great efficacy in the reduction of intestinal disorders caused by different stimulus.

Accordingly, the present invention relates to the use of a compound of formula (I) or a tautomer, a pharmaceutically acceptable salt or a solvate thereof: 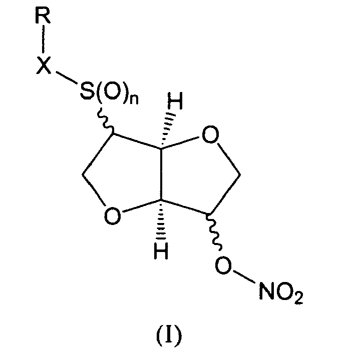 wherein:
n is an integer selected from 0, 1 and 2;
X is -S(O)ₘ-, -(C=O)- or a single bond, wherein m is an integer selected form 0, 1 and 2, with the proviso that when X is -(C=O)- then n is 0;
R is hydrogen or a residue R^{a}, wherein R^{a} is selected from the group consisting of:
   C₁₋₆ alkyl;
   C₂₋₆ alkenyl;
   C₃₋₈ cycloalkyl;
   C₃₋₈ cycloalkyl, wherein one CH₂ group is replaced by O, S, NH or NCH₃;
   C₄₋₈ cycloalkenyl;
   phenyl;
   pyridyl;
   thiophenyl;
   nitrosyl;
   S-cysteinyl;
   S-glutathionyl; and 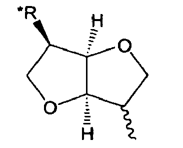 wherein R* is selected from the group consisting of hydrogen, C₁₋₆ alkyl, C₂₋₆ alkenyl, C₃₋₈ cycloalkyl, C₄₋₈ cycloalkenyl, acetyloxy, hydroxyl, ONO₂ and halogen;
   and wherein R^{a} is optionally substituted by one to three groups independently selected from C₁₋₆ alkyl, C₂₋₆ alkenyl, C₃₋₈ cycloalkyl, C₄₋₈ cycloalkenyl, acetyloxy, hydroxyl, ONO₂ and halogen,
as active ingredient(s) in the manufacture of a pharmaceutical composition for the prevention and/or treatment of intestinal disorders.

In a particular embodiment, the intestinal disorder is intestinal inflammation.

In another aspect, the present invention refers to a compound of formula (I) as defined above for use in the treatment or prophylaxis of an intestinal disorder.

### DETAILED DESCRIPTION OF THE INVENTION

In the above definition of compounds of formula (I) used in the present invention, the following terms have the meaning indicated:
"C₁₋₆ alkyl" as used herein refers to a straight or branched hydrocarbon chain radical consisting of carbon and hydrogen atoms, containing no insaturation, having one to six carbon atoms, and which is attached to the rest of the molecule by a single bond, e. g., methyl, ethyl, *n*-propyl, *i*-propyl, *n*-butyl, *t*-butyl, *n*-pentyl.
"C₂₋₆ alkenyl" as used herein refers to a straight or branched chain alkenyl moiety consisting of carbon and hydrogen atoms, having one to six carbon atoms and at least one double bond of either E or Z stereochemistry where applicable, e.g., vinyl, allyl, 1- and 2-butenyl, and 2-methyl-2-propenyl.
"C₃₋₈ cycloalkyl" as used herein refers to an alicyclic group consisting of carbon and hydrogen atoms, having three to eight carbon atoms, e.g., cyclopropyl, cyclobutyl, cyclopentyl and cyclohexyl.

Accordingly, the term "C₃₋₈ cycloalkyl wherein one CH₂ group is replaced by O, S, NH or NCH₃" as used herein refers to an alicyclic group having from three to eight carbon atoms wherein one CH₂ group is replaced by O, S, NH o NCH₃, e.g., tetrahydropyrane, tetrahydrofurane, pyrrolidine, piperidine and tetrahydrothiophene.

"C₄₋₈ cycloalkenyl" as used herein refers to an alicyclic group consisting of carbon and hydrogen atoms, having four to eight carbon atoms, e.g., cyclopentenyl, cyclohexenyl, cycloheptenyl and cyclooctenyl.

"Halogen" as used herein refers to fluorine, chlorine, bromine or iodine, whereof bromine is preferred.

It is preferred that R represents hydrogen C₁₋₆ alkyl, C₂₋₆ alkenyl, C₃₋₈ cycloalkyl, C₄₋₈ cycloalkenyl, (C₁₋₆ alkyl)C₃₋₈ cycloalkyl, (C₁₋₆ alkyl)C₄₋₈ cycloalkenyl, phenyl or (C₁₋₆ alkyl)phenyl, whereas C₁₋₆ alkyl is especially preferred.

It is further preferred that in formula (I) either one or both of m and n is 0.

Also it is preferred that X represents a single bond or -S-.

It is especially preferred that in the compounds of formula (I), RXS(O)ₙ- and ONO₂ are trans to each other with respect to the ring plane. The compound of formula (I) also include (R) and (S) diastereoisomers according to the formula (Ia) and (Ib):

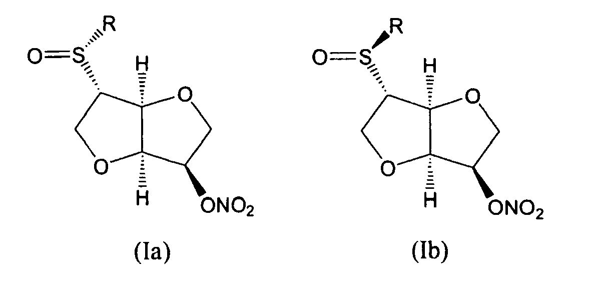

Especially preferred compounds of formula (I) are:
2-thioisosorbide 5-mononitrate;
5,5'-dinitrate-2,2'-dithiodiisosorbide;
2-methylthioisosorbide 5-mononitrate;
2-[(R)-methylsulfinyl]isosorbide 5-mononitrate;
2-[(S)-methylsulfinyl]isosorbide 5-mononitrate;
2-methylsulfinylisosorbide 5-mononitrate;
2-methylsulfonylisosorbide 5-mononitrate;
S-nitroso-2-thioisosorbide 5-mononitrate;
2-(tetrahydropyran-2-yl-thio) isosorbide 5-mononitrate;
2-(isosorbidyl-2'-dithio) isosorbide 5-mononitrate; and
2-(5'-acetyloxyisosorbidyl-2'-dithio) isosorbide 5-mononitrate.

Further it is especially preferred to use 2-acetylthio-isosorbide-5-mononitrate: 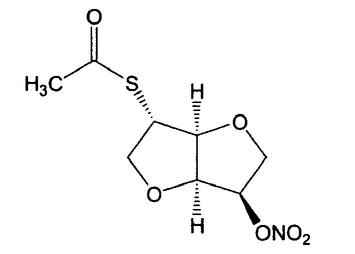 a tautomer, a pharmaceutically acceptable salt or a solvate thereof as active ingredient for the manufacture of a pharmaceutical composition for the prevention and/or treatment of an intestinal disorder.

In a particular embodiment the intestinal disorder is intestinal inflammation.

Unless otherwise stated, the compounds used in the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The term "pharmaceutically acceptable salts, solvates" refers to any pharmaceutically acceptable salt, ester, solvate, or any other compound which, upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. The preparation of salts and derivatives can be carried out by methods known in the art.

For instance, pharmaceutically acceptable salts of compounds used in the invention are synthesized from the parent compound which contains a basic or acidic moiety by conventional chemical methods. Generally, such salts are, for example, prepared by reacting the free acid or base forms of these compounds with a stoichiometric amount of the appropriate base or acid in water or in an organic solvent or in a mixture of the two. Generally, nonaqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate. Examples of the alkali addition salts include inorganic salts such as, for example, sodium, potassium, calcium, ammonium, magnesium, aluminium and lithium salts, and organic alkali salts such as, for example, ethylenediamine, ethanolamine, N,N-dialkylenethanolamine, triethanolamine, glucamine and basic aminoacids salts.

Particularly favoured derivatives or prodrugs are those that increase the bioavailability of the compounds of this invention when such compounds are administered to a patient (e.g., by allowing an orally administered compound to be more readily absorbed into the blood) or which enhance delivery of the parent compound to a biological compartment (e.g., the brain or lymphatic system) relative to the parent species.

The compounds used in the present invention may be in crystalline form either as free compounds or as solvates (e.g. hydrates).

The compounds of formula (I) or their salts or solvates used in the invention are preferably in pharmaceutically acceptable or substantially pure form. By pharmaceutically acceptable form is meant, "inter alia", having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I), or of its salts or, solvates.

The compounds used in the present invention represented by the formula (I) can include enantiomers, depending on the presence of chiral centers, and/or depending on the presence of multiple bonds (for example Z, E). The pure isomers, enantiomers or diastereoisomers and their mixtures are within the scope of the present invention.

The compounds of formula (I) used in the invention can be obtained by available synthetic procedures. Some examples of these procedures are described in W02005/037842 and references therein.

In a particular embodiment of the present invention, the compounds of formula (I) for the treatment of intestinal disorders, are formulated in a suitable pharmaceutical composition, in a therapeutically effective quantity, together with one or more pharmaceutically acceptable carriers, adjuvants or excipients.

The pharmaceutical composition may be administered in the form of different preparations. Non limiting examples are preparations for oral administration, e.g. tablets, capsules, syrups or suspensions; ophthalmological administration, e.g. solutions, suspensions, ointments or creams; and parenteral administration, e.g. aqueous and non-aqueous sterile injection solutions or aqueous and non-aqueous sterile suspensions. Also, the pharmaceutical composition may include topical compositions, e.g. creams, ointments or pastes, or transdermic preparations such as patches or plasters. The pharmaceutical composition may also be prepared for vaginal or for rectal administration, e.g. rectal gel or suppository.

Generally an effective administered amount of a compound used in the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.01 to 100 mg/kg/day.

The compounds used in the present invention may also be administered with other drugs to provide a combination therapy. The other drugs may form part of the same composition, or be provided as a separate composition for administration at the same time or at different time.

In another particular embodiment the invention refers to the use of a compound of formula (I) or a tautomer, a pharmaceutically acceptable salt, a prodrug or a solvate thereof, for the manufacture of a pharmaceutical composition for the treatment and/or prevention of an intestinal disorder. In a preferred embodiment, the intestinal disorder is an intestinal inflammation.

*In vivo* experiments in animals subjected to different stimulus such as high intestinal inflammation have shown that administration of compounds of formula (I), reduce significantly these adverse effects. Therefore, these compounds have a great efficacy in the reduction of intestinal disorders caused by different stimulus.

### EXAMPLES

### Example 1.- Results derived from the administration of 2-acetylthioisosorbide-5-mononitrate in mice which have been subjected to indomethacin-induced intestinal inflammation.

Intestinal inflammation were induced by administration of two subcutaneous injections of 7.5 mg/kg indomethacin, as previously described Porras M., Martin MT., Soler M. and Vergara P., "Intestinal motor disorders associated with cyclical bacterial overgrowth in a rat model of enteritis". Am. J. Physiol. Gastrointest. Liver Physiol. (2004), 287:G58-G64, and Porras M., Martin MT., Torres R. and Vergara P., "Cyclical upregulated iNOS and long-term downregulated nNOS are the bases for relapse and quiescent phases in a rat model of IBD". Am. J. Physiol. Gastrointest. Liver Physiol. (2006), 290:G423-G430.

Once the intestinal inflammation has been induced, one group of mice were treated with 30 mg of the compound 2-acetylthioisosorbide-5-mononitrate per kg of body weight, being dissolved this compound in water and administered orally. For comparative data, the compound isosorbide-5-mononitrate (IS-5-MN), structurally similar to 2-acetylthioisosorbide-5-mononitrate, was also administered to another group of mice.

Myeloperoxidase (MPO) activity was measured as an inflammation index in homogenized tissue samples after centrifugation using a specific ELISA kit (HyCult biotechnology, Uden, The Netherlands). In addition, since the barrier function of the intestinal mucus is deteriorated when the epithelium is inflamed and, as a consequence, the exposed bacteria cross the membrane and enter in the blood current, bacterial translocation was measured by detection of viable enteric bacteria in mesenteric lymph nodes as described by [M Mainous MR., Tso P., Berg R.D. and Deitch E.A., Arch Surg (1991) 126:33-37] as additional measure of inflammation degree.

Studies of the route, magnitude, and time course of bacterial translocation in a model of systemic inflammation were achieved as describe in Arch Surg 126:33-37, 1991. Bacterial translocation was expressed as the number of positive cultures with respect to the total number of samples in each group and motor activity was measured as the contracting activity expressed as the total number of spontaneous contractions recorded at duodenum per minute. Results are given in Table 1.

**Table 1**

| Group | Bacterial translocation | | MPO activity | Contractions/min |
|---|---|---|---|---|
| | E. coli | Enterococcus sp | ng/ml | |
| Control | 1/6 | 0/6 | 5±1 | 0.25±0.1 |
| Intestinal inflammation | 4/6 | 4/6 | 25±2 | 0.6±0.1 |
| IS-5-MN | 3/6 | 3/6 | 19±4 | 0.5±0.1 |
| 2-acetyl thioisosorbide-5-mononitrate | 2/6 | 1/6 | 10±2* | 0.2±0.1 |

These results point out that compound 2-acetylthioisosorbide-5-mononitrate leads to a significant reduction of MTO activity when compared to a structurally similar compound. In addition, the bacterial translocation is also reduced avoiding the entry of bacteria to the blood current, therefore confirming the utility of the compounds of the invention for treating intestinal inflammation.

## Claims

1. A compound of formula (I) or a tautomer, a pharmaceutically acceptable salt or a solvate thereof: 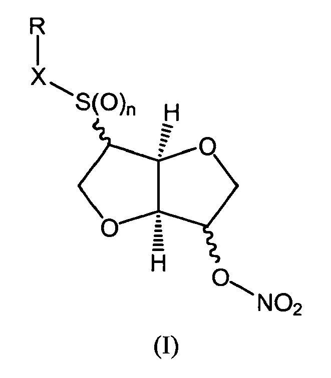 wherein:
n is an integer selected from 0, 1 and 2;
X is -S(O)m-, -(C=O)- or a single bond, wherein m is an integer selected form 0, 1 and 2, with the proviso that when X is -(C=O)- then n is 0;
R is hydrogen or a residue Ra, wherein Ra is selected from the group consisting of:
C₁₋₆ alkyl;
C₂₋₆ alkenyl;
C₃₋₈ cycloalkyl;
C₃₋₈ cycloalkyl, wherein one CH₂ group is replaced by O, S, NH or NCH₃;
C₄₋₈ cycloalkenyl;
phenyl;
pyridyl;
thiophenyl;
S-cysteinyl;
S-glutathionyl; and 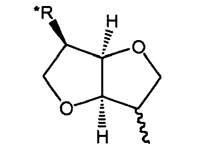 wherein R* is selected from the group consisting of hydrogen, C₁₋₆ alkyl, C₂₋₆ alkenyl, C₃₋₈ cycloalkyl, C₄₋₈ cycloalkenyl, acetyloxy, hydroxyl, ONO₂ and halogen;
and wherein R^{a} is optionally substituted by one to three groups independently selected from C₁₋₆ alkyl, C₂₋₆ alkenyl, C₃₋₈ cycloalkyl, C₄₋₈ cycloalkenyl, acetyloxy, hydroxyl, ONO₂ and halogen,
for use in the prevention and/or treatment of intestinal inflammation.

2. A compound according to claim 1, wherein either one or both of m and n is 0.

3. A compound according to any one of claims 1 or 2, wherein X represents a single bond or -S-.

4. A compound according to any one of claims 1 to 3, wherein R is hydrogen C₁₋₆ alkyl, C₂₋₆ alkenyl, C₃₋₈ cycloalkyl, C₄₋₈ cycloalkenyl, (C₁₋₆ alkyl)C₃₋₈ cycloalkyl, (C₁₋₆ alkyl)C₄₋₈ cycloalkenyl, phenyl or (C₁₋₆ alkyl)phenyl.

5. A compound according to any one of claims 1 to 4, wherein R is C₁₋₆ alkyl.

6. A compound according to any one of claims 1 to 5, wherein the compound according to formula (I) is a compound of formula (Ia) or (Ib):

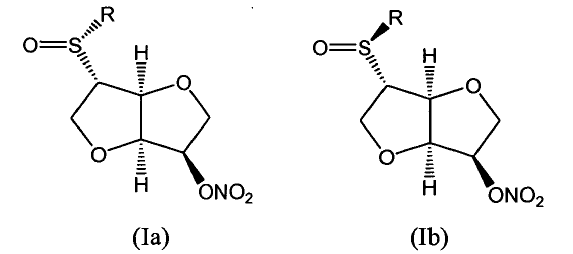

7. A compound according to claim 1, wherein the compound of formula (I) is selected from:
2-thioisosorbide 5-mononitrate;
5,5'-dinitrate-2,2'-dithiodiisosorbide;
2-methylthioisosorbide 5-mononitrate;
2-[(R)-methylsulfinyl]isosorbide 5-mononitrate;
2-[(S)-methylsulfinyl]isosorbide 5-mononitrate;
2-methylsulfinylisosorbide 5-mononitrate;
2-methylsulfonylisosorbide 5-mononitrate;
S-nitroso-2-thioisosorbide 5-mononitrate;
2-(tetrahydropyran-2-yl-thio) isosorbide 5-mononitrate;
2-(isosorbidyl-2'-dithio) isosorbide 5-mononitrate; and
2-(5'-acetyloxyisosorbidyl-2'-dithio) isosorbide 5-mononitrate.

8. A compound according to claim 1, wherein the compound is 2-acetylthioisosorbide 5-mononitrate which is represented by the following formula:

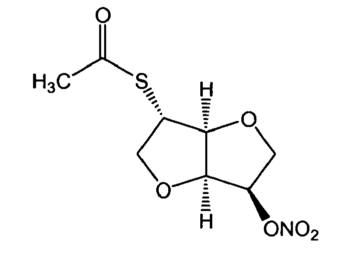

9. The use of a compound of formula (I) as defined in any of claims 1 to 8 as active ingredient in the manufacture of a pharmaceutical composition for the treatment and/or prophylaxis of intestinal inflammation.

## Patentansprüche

1. Verbindung der Formel (I) oder ein Tautomer, ein pharmazeutisch akzeptables Salz oder ein Solvat davon: 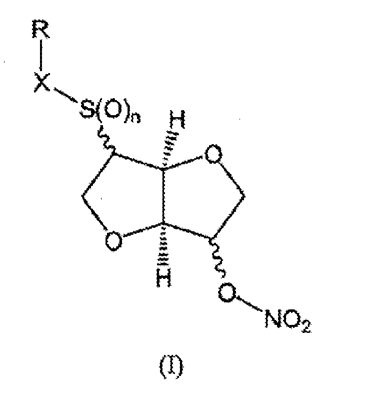 wobei:
n eine ganze Zahl aus 0, 1 und 2 ist;
X-S(O)ₘ₋, -(C=O)- oder eine Einzelbindung ist, wobei m ein ganze Zahl aus 0, 1 und 2 ist, vorausgesetzt, dass, wenn X -(C=O)- ist, dann ist n = 0;
R Wasserstoff oder ein Rest R^{a} ist, wobei R^{a} aus der Gruppe ausgewählt ist:
C₁₋₆-Alkyl;
C₂₋₆-Alkenyl;
C₃₋₈-Cycloalkyl;
C₃₋₈-Cycloalkyl, wobei eine CH₂-Gruppe durch O, S, NH oder NCH₃ ersetzt ist;
C₄₋₈-Cycloalkenyl;
Phenyl;
Pyridyl;
Thiophenyl;
Nitrosyl;
S-Cysteinyl;
S-Glutathionyl; und 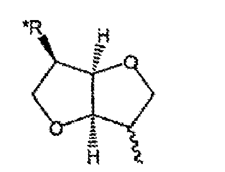
wobei R* aus der Gruppe ausgewählt ist: Wasserstoff C, C₁₋₆-Alkyl, C₂₋₆-Alkenyl, C₃₋₈-Cycloalkyl, C₄₋₈-Cycloalkenyl, Acetyloxy, Hydroxyl, ONO₂ und Halogen;
und wobei R^{a} wahlweise ersetzt wird durch ein bis drei Gruppen, die unabhängig ausgewählt sind aus C₁₋₆-Alkyl, C₂₋₆-Alkenyl, C₃₋₈-Cycloalkyl, C₄₋₈-Cycloalkenyl, Acetyloxy, Hydroxyl, ONO₂ und Halogen,
für die Verwendung zur Prävention und/oder Behandlung von Darmentzündung.

2. Verbindung nach Anspruch 1 oder 2, wobei entweder m oder n oder beides 0 ist.

3. Verbindung nach einem der Ansprüche 1 oder 2, wobei X eine Einfachbindung oder -S- repräsentiert.

4. Verbindung nach einem der Ansprüche 1 bis 3, wobei R Wasserstoff, ein C₁₋₆-Alkyl, C₂₋₆-Alkenyl, C₃₋₈-Cycloalkyl, C₄₋₈-Cycloalkenyl, (C₁₋₆-Alkyl)C₃-₈-Cykloalkyl, (C₁₋₆-Alkyl)C₄-₈-Cycloalkenyl, Phenyl oder (C₁₋₆-Alkyl)Phenyl ist.

5. Verbindung nach einem der Ansprüche 1 bis 4, wobei R ein C₁₋₆-Alkyl ist.

6. Verbindung nach einem der Ansprüche 1 bis 5, wobei die Verbindung gemäß der Formel (I) eine Verbindung gemäß der Formel (Ia) oder (Ib) ist:

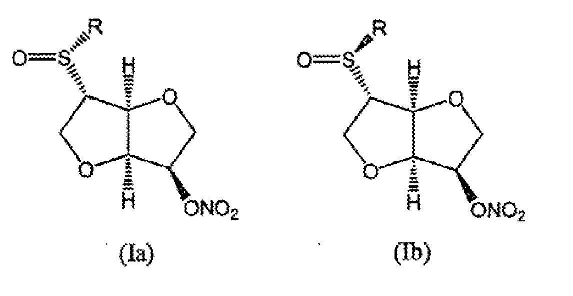

7. Verbindung nach Anspruch 1, wobei die Verbindung der Formel (I) ausgewählt ist aus:
2-Thioisosorbid-5-Mononitrat;
5,5'-Dinitrat-2,2'-Dithiodiisosorbid;
2-Methylthioisosorbid-5-Mononitrat;
2-[(R)-Methylsulfinyl]Isosorbid-5-Mononitrat;
2-[(S)-Methylsulfinyl]Isosorbid-5-Mononitrat;
2-Methylsulfinylisosorbid-5-Mononitrat;
2-Methylsufonylisosorbid-5-Mononitrat;
S-Nitroso-2-Thioisosorbid-5-Mononitrat;
2-(Tetrahydropyran-2-yl-thio)Isosorbid-5-Mononitrat;
2-(Isosorbidyl-2'-Dithio)lsosorbid-5-Mononitrat; und
2-(5'-Acetyloxyisosorbidyl-2'-Dithio)Isosorbid-5-Mononitrat.

8. Verbindung nach Anspruch 1, wobei die Verbindung 2-Acetylthioisosorbid-5-Mononitrat ist, das durch die folgende Formel repräsentiert wird:

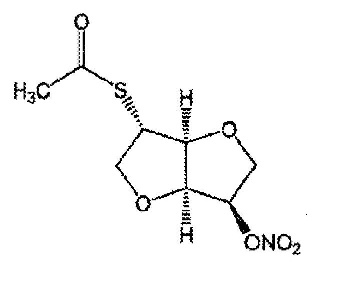

9. Verwendung einer Verbindung der Formel (I), wie in einem der Ansprüche 1 bis 8 definiert, als Wirkstoff bei der Herstellung einer pharmazeutischen Zusammensetzung für die Behandlung und/oder Prophylaxe von Darmentzündung.

## Revendications

1. Composé de formule (I) ou tautomère, sel pharmaceutiquement acceptable ou solvate de celui-ci : 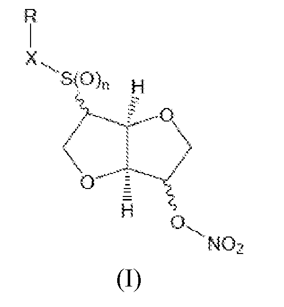 où :
n est un nombre entier sélectionné parmi 0, 1 et 2 ;
X est -S(O)ₘ-, -(C=O)- ou une liaison simple, où m est un nombre entier sélectionné parmi 0, 1 et 2, à condition que lorsque X est -(C=O)- n soit alors égal à 0;
R est un atome d'hydrogène ou un résidu R^{a}, dans lequel R^{a} est sélectionné dans le groupe :
alkyle en C₁₋₆ ;
alcényle en C₂₋₆ ;
cycloalkyle en C₃₋₈ ;
cycloalkyle en C₃₋₈, dans lequel un groupe CH₂ est remplacé par O, S,
NH ou NCH₃ ;
cycloalcényle en C₄₋₈ ;
phényle ;
pyridyle ;
thiophényle ;
nitrosyle ;
S-cystéinyle ;
S-glutathionyle ; et 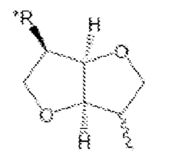 où R* est sélectionné dans le groupe: un atome d'hydrogène, un groupe alkyle en C₁₋₆, alcényle en C₂₋₆, cycloalkyle en C₃₋₈, cycloalcényle en C₄₋₈, acétyloxy, hydroxyle, ONO₂ et un atome d'halogène;
et où R^{a} est facultativement substitué par un à trois groupes sélectionnés indépendamment parmi un groupe alkyle en C₁₋₆, alcényle en C₂₋₆, cycloalkyle en C₃₋₈, cycloalcényle en C₄₋₈, acétyloxy, hydroxyle, ONO₂ et un atome d'halogène,
pour l'utilisation dans la prévention et/ou le traitement de la inflammation intestinale.

2. Composé selon la revendication 1, dans lequel soit m, soit n, soit les deux, est égal à 0.

3. Composé selon l'une quelconque des revendications 1 à 2, dans lequel X représente une liaison simple ou -S-.

4. Composé selon l'une quelconque des revendications 1 à 3, dans lequel R est hydrogène, alkyle en C₁₋₆, alcényle en C₂₋₆, cycloalkyle en C₃₋₈, cycloalcényle en C₄₋₈, (alkyle en C₁₋₆)-cycloalkyle en C₃₋₈, (alkyle en C₁₋₆)-cycloalcényle en C₄₋₈, phényle ou (alkyle en C₁₋₆)phényle.

5. Composé selon l'une quelconque des revendications 1 à 4, dans lequel R est un groupe alkyle en C₁₋₆.

6. Composé selon l'une quelconque des revendications 1 à 5, dans lequel le composé selon la formule (I) est un composé de formule (Ia) ou (Ib) :

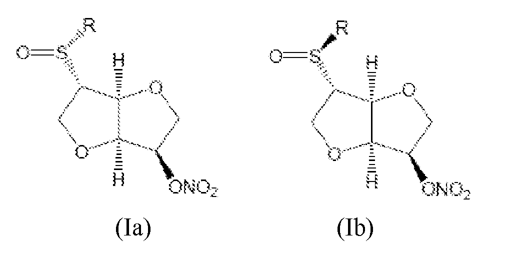

7. Composé selon la revendication 1, dans lequel le composé de formule (I) est sélectionné parmi :
le 5-mononitrate de 2-thioisosorbide ;
le 5,5'-dinitrate-2, 2'-dithiodiisosorbide ;
le 5-mononitrate de 2-méthylthioisosorbide ;
le 5-mononitrate de 2-[(R)-méthylsulfinyl]isosorbide ;
le 5-mononitrate de 2-[(S)-méthylsulfinyl]isosorbide ;
le 5-mononitrate de 2-méthylsulfinylisosorbide ;
le 5-mononitrate de 2-méthylsulfonylisosorbide ;
le 5-mononitrate de S-nitroso-2-thioisosorbide ;
le 5-mononitrate de 2-(tétrahydropyran-2-yl-thio)isosorbide ;
le 5-mononitrate de 2-(isosorbidyl-2'-dithio)isosorbide ; et
le 5-mononitrate de 2-(5'-acétyloxyisosorbidyl-2'-dithio)isosorbide.

8. Composé selon la revendication 1, dans lequel le composé est le 5-mononitrate de 2-acétylthioisosorbide qui est représenté par la formule suivante :

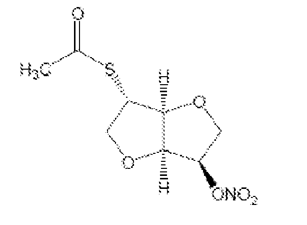

9. Utilisation d'un composé de formule (I) selon l'une quelconque des revendications 1 à 8, en tant que principe actif dans la fabrication d'une composition pharmaceutique destinée au traitement et/ou à la prophylaxie de la inflammation intestinale.
